# FASCICULE DE BREVET EUROPEEN

(11) **EP 3 268 378 B1**
(45) Date de publication et mention de la délivrance du brevet: **26.12.2018**
(21) Numéro de dépôt: 16712274.6
(22) Date de dépôt: 11.03.2016
(51) Int. Cl.: C07H 15/06, A61K 8/60, A61Q 17/00, A61Q 19/00

(54) **GALACTOLIPIDE POUR SON UTILISATION DANS LA CICATRISATION ET DANS LA PRÉVENTION ET/OU LE TRAITEMENT DU VIEILLISSEMENT CUTANÉ**
GALACTOLIPID ZUR ANWENDUNG IN WUNDHEILUNG UND ZUR BEHANDLUNG UND/ODER PREVENTION VON HAUTALTERUNG
GALACTOLIPID FOR USE IN WOUND HEALING AND FOR USE IN THE PREVENTION AND/OR TREATMENT OF SKIN AGING

(30) Priorité: 11.03.2015 FR 1552028
(43) Date de publication de la demande: 17.01.2018
(73) Titulaire: Pierre Fabre Dermo-Cosmétique, 92100 Boulogne-Billancourt (FR)
(72) Inventeur: MANDEAU, Anne, 31200 Toulouse (FR); HERNANDEZ-PIGEON, Hélène, 31270 Cugnaux (FR); DUPLAN, Hélène, 31320 Auzeville Tolosan (FR)
(74) Mandataire: Regimbeau
(86) Numéro de dépôt international: PCT/EP2016/055324
(87) Numéro de publication internationale: WO 2016/142529

(56) Documents cités:
- EP-A1- 1 043 016
- WO-A1-96/25142
- MATS HAMBERG ET AL: "Isolation and structure of a new galactolipid from oat seeds", LIPIDS, vol. 33, no. 4, 1 avril 1998 (1998-04-01), pages 355-363, XP055198199, ISSN: 0024-4201, DOI: 10.1007/s11745-998-0215-9 cité dans la demande
- ROBERT A. MOREAU ET AL: "The Identification of Mono-, Di-, Tri-, and Tetragalactosyl-diacylglycerols and their Natural Estolides in Oat Kernels", LIPIDS, vol. 43, no. 6, 15 mai 2008 (2008-05-15), pages 533-548, XP055198197, ISSN: 0024-4201, DOI: 10.1007/s11745-008-3181-6 cité dans la demande
- K. BINIEK ET AL: "Solar UV radiation reduces the barrier function of human skin", PROCEEDINGS OF THE NATIONAL ACADEMY OF SCIENCES, vol. 109, no. 42, 16 octobre 2012 (2012-10-16), pages 17111-17116, XP055198203, ISSN: 0027-8424, DOI: 10.1073/pnas.1206851109 cité dans la demande
- MARCIA RAMOS-E-SILVA ET AL: "Effects of age (neonates and elderly) on skin barrier function", CLINICS IN DERMATOLOGY, vol. 30, no. 3, 1 mai 2012 (2012-05-01), pages 274-276, XP055198201, ISSN: 0738-081X, DOI: 10.1016/j.clindermatol.2011.08.024 cité dans la demande

## Description

La présente invention concerne notamment l'utilisation de galactolipides particuliers pour la cicatrisation, pour la réparation, le renforcement et le rééquilibre de la barrière cutanée dont la barrière mécanique, l'hydratation cutanée, et pour la prévention et le traitement du vieillissement cutané.

Alors que les composés majoritaires de la plupart des membranes biologiques sont les phospholipides, les lipides les plus abondants dans les chloroplastes sont les galactolipides, MGDG et DGDG (mono- et di-galactosyldiacylglycerols). En plus des chloroplastes, ces galactolipides sont présents en grosse quantité dans les grains d'avoine. Hamberg *et al.* (1996) ont mis en évidence dans les grains d'avoine la présence d'un acide gras hydroxylé particulier, l'acide 15(*R*)-hydroxylinoléique, ou acide avenoléique, et plus tard, la présence d'un nouveau galactolipide consistant en l'assemblage d'une molécule d'acide avenoléique (hydroxyacide), deux molécules d'acide linoléique, deux molécules de D-galactose et une molécule de glycérol (Hamberg *et al.* (1998)). Cette molécule est présente à hauteur de 0,5-0,6 mg / g de graines, équivalent à la quantité des autres digalactosyldiacylglycérols contenant des chaines acylées non oxygénées.

L'acide avenoléique est une oxylipine (acide 15(R)-hydroxy-(9Z),(12Z)-octadecadiénoïque) présent dans l'avoine mais non détectable dans les grains d'orge, de seigle et de blé. Il est proche chimiquement de l'acide ricinoléique. Il est stocké dans les graines sous forme de galactolipides. Les galactolipides contenant des acides gras oxygénés sont rares. Celui-ci est un estolide naturel, un estolide étant formé lorsqu'un acide gras hydroxylé est estérifié par un autre acide gras (Doehlert *et al.* (2010)).

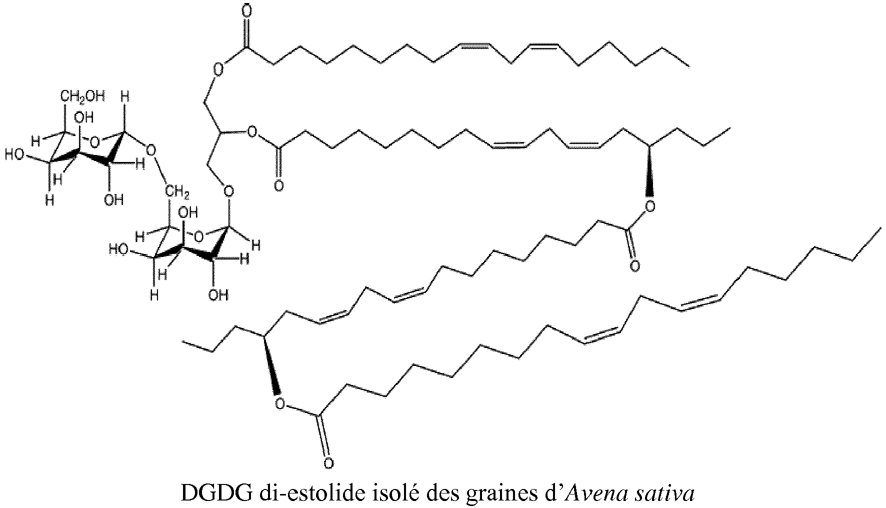

Le DGDG mono-estolide est le plus abondant. Il est accompagné par deux DGDG di- et tri-estolides, deux trigalactosyldiacylglycerol mono- et di-estolides et un tetragalactosyldiacylglycerol mono-estolide.

Les estolides naturels sont cependant rares. Des triacylglycerol estolides ont été décrits dans les huiles de graines de 3 espèces de *Sebastiana,* dans l'huile d'ergot, dans l'huile de *Lesquerella* et dans l'huile de ricin. Aucun estolide de digalactosyl diacylglycérol (DGDG) n'a été décrit à ce jour en dehors de ceux isolés de la graine d'avoine (Moreau *et al.* (2008)).

Les demandes de brevet WO96/25142 et EP1043016 décrivent l'activité de galactolipides, en particulier de galactosylglycérides et des extraits naturels les contenant, sur la production extracellulaire de fibronectine par les fibroblastes humains, amplifiant ainsi le réseau extracellulaire de fibronectine, et leur utilisation pour prévenir ou traiter les les effets du vieillissement et favoriser la cicatrisation. Aucune activité particulière des estolides de DGDG n'a cependant été décrite.

Les inventeurs de la présente invention ont découvert de manière surprenante que des galactolipides de type DGDG étaient utiles dans la cicatrisation cutanée et pouvaient ainsi être utilisés, notamment par voie topique, dans la réparation, le renforcement et le rééquilibre de la barrière cutanée.

Ils ont également démontré l'effet des galactolipides de type DGDG sur le renforcement et le rééquilibre de la barrière mécanique de la peau, ainsi que sur l'hydratation cutanée, en particulier l'hydratation du stratum corneum (SC). L'hydratation cutanée peut être améliorée par une meilleure captation et rétention de molécules d'eau dans le stratum corneum, ou par une limitation de la diffusion intra-corporelle vers l'extérieur. Une meilleure hydratation du SC conduit à une amélioration des sensations de tiraillements de la peau et diminution des rugosités, craquelures et/ou crevasses.

Les inventeurs ont utilisé un modèle *in vitro* de SC isolé permettant d'étudier les modifications de ses propriétés mécaniques en fonction de son niveau d'hydratation. Les paramètres mesurés sont le module d'élasticité, attestant de la rigidité du tissu à l'extension, la tension cutanée et l'énergie de fissuration. L'association de ces paramètres permet de définir une relation entre l'état de sécheresse cutanée et le niveau de contrainte cutanée ainsi que l'apparition de craquelures. En outre, l'utilisation de ce modèle permet de connaître avec une grande précision l'impact d'actifs sur ces mesures mécaniques, et par conséquent, sur l'état d'hydratation du stratum corneum. (Levi *et al.* (2010) ; Vyumvuhore et al. 2015).

Par « barrière cutanée », on entend, au sens de la présente invention, la protection conférée par la couche épidermique de la peau. Cette « barrière » assure l'étanchéité de l'organisme et elle consiste plus particulièrement à protéger l'organisme de la déshydratation dans un milieu non aquatique, à la protéger de stresses ou d'agressions environnementales.

La barrière cutanée, au sens de la présente invention, inclut donc la barrière mécanique conférée par la peau, et plus particulièrement par le stratum corneum. Cette barrière mécanique peut être évaluée notamment selon l'approche biomécanique du SC (évaluation de l'état de contrainte du SC isolé déshydraté décrite dans Levi *et al.* (2010)).

Avantageusement, l'enseignement de la présente invention s'adresse aux peaux fragiles. En effet, il est décrit que les peaux fragiles présentent de nombreux signes d'une barrière cutanée altérée (Ramos e Silva *et al.* (2013) ; Biniek *et al.* (2012).

Les inventeurs ont aussi mis en évidence que ces galactolipides de type DGDG étaient utiles dans la prévention et le traitement du vieillissement cutané, dont le photovieillissement cutané (vieillissement de la peau induit par les rayons UV, notamment du soleil).

La présente invention a donc pour objet un galactolipide de formule (I) suivante : 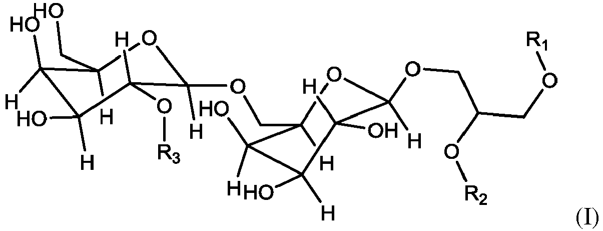 dans laquelle :
- R₁ et R₂, identiques ou différents, représentent un groupe -CO-R₄, 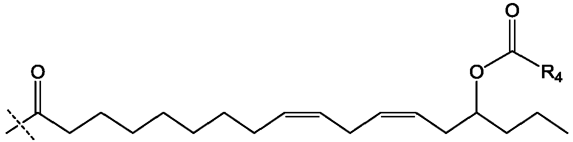 ou 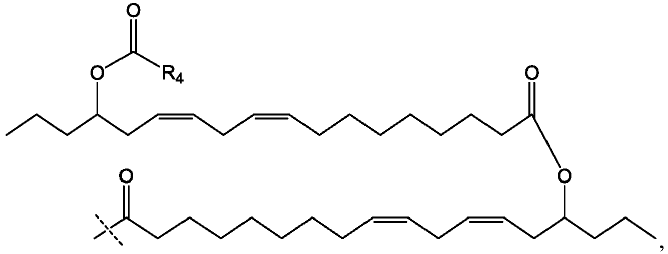
- R₃ représente :
   ▪ un groupe -CO-R₅ lorsque R₁ et R₂ représentent chacun, indépendamment l'un de l'autre, un groupe -CO-R₄,
   ▪ un atome d'hydrogène ou un groupe -CO-R₅ lorsqu'au moins R₁ ou R₂ représente un groupe autre que -CO-R₄, et
- R₄ et R₅, identiques ou différents, représentent une chaîne hydrocarbonée linéaire ou ramifiée, de préférence linéaire, saturée ou insaturée, comprenant 9 à 25, notamment 11 à 21, en particulier 13 à 19, plus particulièrement 15 à 17, atomes de carbone, éventuellement substituée par un groupe OH,
pour son utilisation dans la cicatrisation, de préférence de la peau ; la réparation, le renforcement et le rééquilibre de la barrière cutanée ; et la prévention et le traitement du vieillissement cutané, en particulier la prévention et le traitement du photovieillissement cutané.

La présente invention concerne également l'utilisation d'un galactolipide de formule (I), pour la préparation d'une composition dermo-cosmétique ou dermatologique utile dans la cicatrisation, de préférence de la peau ; la réparation, le renforcement et le rééquilibre de la barrière cutanée dont le renforcement et le rééquilibre de la barrière mécanique de la peau ; l'hydratation cutanée, en particulier l'hydratation cutanée du stratum corneum ; et la prévention et le traitement du vieillissement cutané, en particulier la prévention et le traitement du photovieillissement cutané.

La présente invention concerne également l'utilisation d'un galactolipide de formule (I), pour la cicatrisation, de préférence de la peau ; la réparation, le renforcement et le rééquilibre de la barrière cutanée dont le renforcement et le rééquilibre de la barrière mécanique de la peau ; l'hydratation cutanée, en particulier l'hydratation cutanée du stratum corneum ; et la prévention et le traitement du vieillissement cutané, en particulier la prévention et le traitement du photovieillissement cutané.

La présente invention concerne également une méthode de cicatrisation, de préférence de la peau ; de réparation, de renforcement et de rééquilibre de la barrière cutanée dont le renforcement et le rééquilibre de la barrière mécanique de la peau ; l'hydratation cutanée, en particulier l'hydratation cutanée du stratum corneum ; ou de prévention ou de traitement du vieillissement cutané, en particulier de prévention ou de traitement du photovieillissement cutané, comprenant l'administration à une personne en ayant besoin d'une quantité efficace d'un galactolipide de formule (I).

Le galactolipide selon l'invention pourra être notamment un galactolipide de formule (I') tel que définie ci-après.

Le galactolipide selon l'invention pourra également être en particulier un galactolipide selon l'une des formules (la) à (Id) ci-dessous : 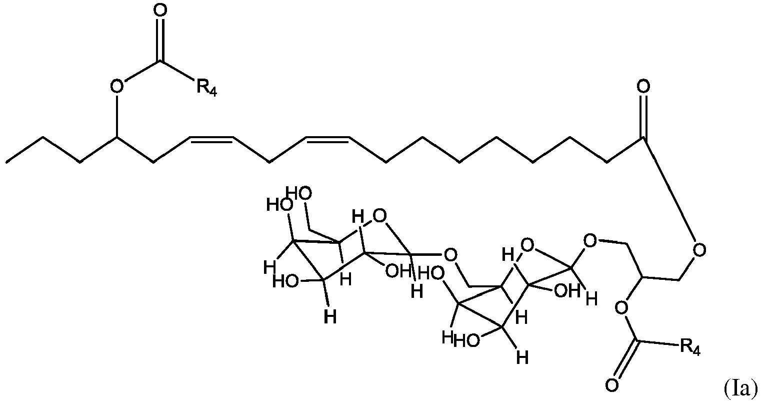 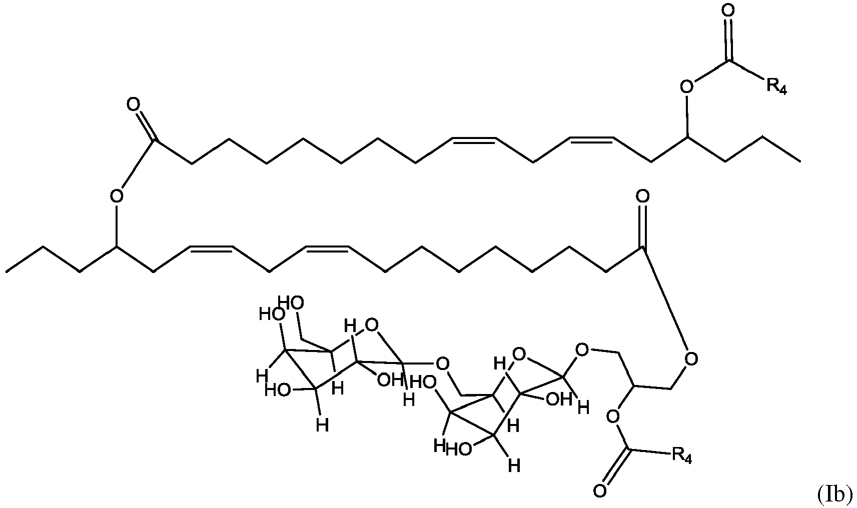 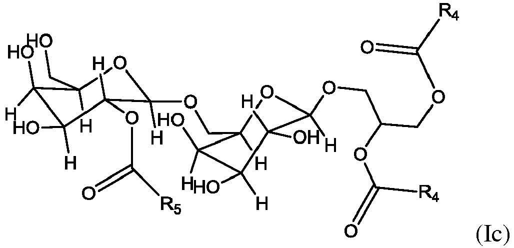 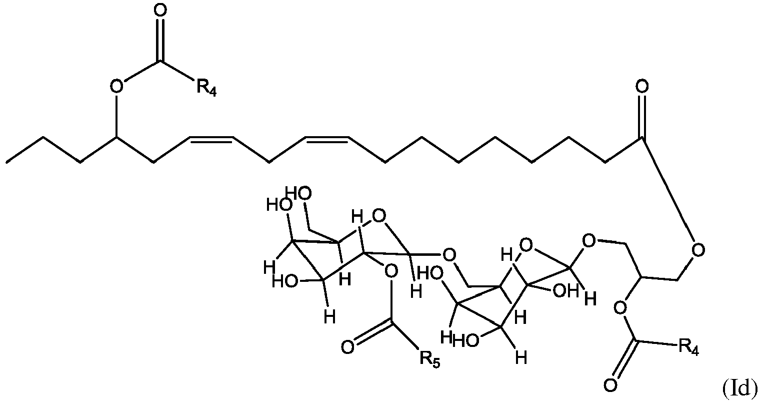 pour lesquelles R₄ et R₅ sont tels que définis ci-dessus et ci-après.
La formule (la) correspond à des DGDG monoestolides.
La formule (Ib) correspond à des DGDG diestolides.
La formule (Ic) correspond à des acylDGDG.
La formule (Id) correspond à des acylDGDG monoestolides.

Les groupes -CO-R₄ et -CO-R₅ correspondent donc à des groupements issus d'acides gras de formules respectives R₄-COOH et R₅-COOH.

R₄ et R₅, identiques ou différents, représentent une chaîne hydrocarbonée linéaire ou ramifiée, de préférence linéaire, saturée ou insaturée, comprenant 9 à 25, notamment 11 à 21, en particulier 13 à 19, plus particulièrement 15 à 17, encore plus particulièrement 15 ou 17 atomes de carbone, éventuellement substituée par un groupe OH, et notamment non substituée.

Avantageusement, R₄ et R₅, identiques ou différents, représentent, une chaîne hydrocarbonée linéaire ou ramifiée, de préférence linéaire, saturée ou comprenant 1 à 5, notamment 1 à 3 doubles liaisons C=C, comprenant 9 à 25, notamment 11 à 21, en particulier 13 à 19, plus particulièrement 15 à 17, encore plus particulièrement 15 ou 17 atomes de carbone, éventuellement substituée par un groupe OH, et notamment non substituée.

De préférence, R₄ et R₅, identiques ou différents, représentent une chaîne hydrocarbonée linéaire, saturée ou comprenant 1 à 3 (en particulier 1 ou 2) doubles liaisons C=C, comprenant 13 à 21, notamment 13 à 19, en particulier 15 à 17, plus particulièrement 15 ou 17, atomes de carbone, éventuellement substituée par un groupe OH, et notamment non substituée.

Plus particulièrement, les groupes R₄ et R₅, identiques ou différents, pourront être choisis parmi les groupes suivants : 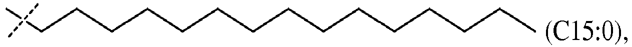 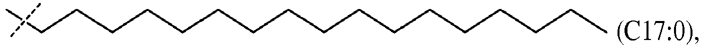 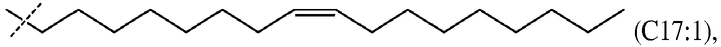 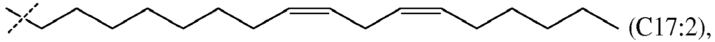 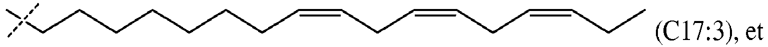 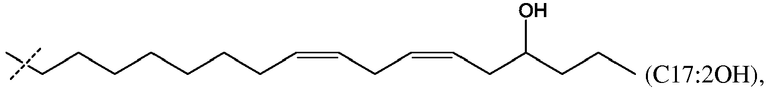 et plus particulièrement parmi les groupes (C15:0), (C17:0), (C17:1), (C17:2), et (C17:3), et encore plus particulièrement parmi les groupes (C15:0), (C17:0), (C17:1) et (C17:2).

Ces groupes particuliers correspondent aux chaînes latérales des acides gras suivants :

### Acide palmitique (C16:0) :

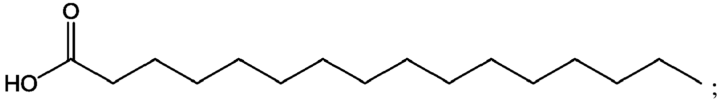

### Acide stéarique (C18:0) :

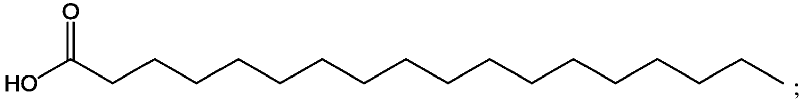

### Acide oléique (C18:1) :

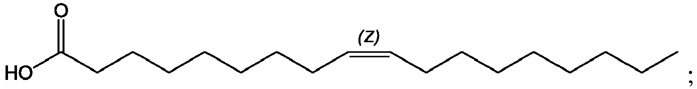

### Acide linoléique (C18:2) :

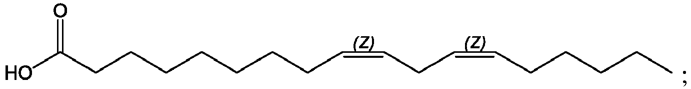

### Acide linolénique (C18:3) :

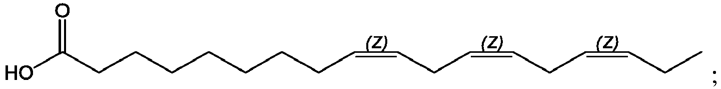

### Acide avénolique (C18:2OH) :

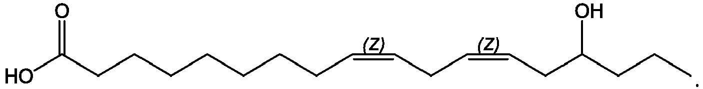

De manière avantageuse, R₄ et R₅ sont identiques.

Le galactolipide selon l'invention pourra être choisi en particulier parmi les galactolipides selon les formules (la) à (Id) ci-dessus avec R₄ et R₅ identiques et choisis parmi les groupes (C15:0), (C17:0), (C17:1), (C17:2), (C17:3) et (C17:2OH), plus particulièrement parmi les groupes (C15:0), (C17:0), (C17:1), (C17:2) et (C17:3) ; et encore plus particulièrement parmi les groupes (C15:0), (C17:0), (C17:1) et (C17:2). Il pourra s'agir en particulier d'un galactolipide de formule (Ia) avec R₄ et R₅ identiques et choisis parmi les groupes (C15:0), (C17:0), (C17:1) et (C17:2), ou de formule (Ib), (Ic) ou (Id) avec R₄ = R₅ = (C17:2).

Le galactolipide selon l'invention est destiné plus particulièrement à être appliqué de manière topique, en particulier sur la peau, et plus particulièrement sur une peau fragile.

Les galactolipides selon la présente invention peuvent être extraits des graines d'avoine ou des graines d'avoine maltée.

Le maltage est une opération bien connue de l'homme du métier qui consiste à reproduire, de façon industrielle, la germination d'une céréale (ici l'avoine). Son principe consiste à créer les conditions idéales de développement du grain (chaleur et humidité) afin qu'il produise certaines enzymes (cytases, amylases, phosphatases, peptidases). Ces enzymes permettront la transformation de l'amidon en sucres simples (notamment en glucose) et à l'embryon de pénétrer à travers la barrière hémicellulosique pour accéder ainsi à ses réserves.

Le maltage se déroule en 4 étapes :
- une phase de trempe durant laquelle le grain est humidifié,
- une phase de germination durant laquelle le grain va germer,
- une phase de touraillage durant laquelle le grain germé est séché à l'air chaud, et
- une phase de dégermination permettant d'éliminer les radicelles.

Les extraits de graines d'avoine ou d'avoine maltée peuvent être obtenus par pression des graines pour obtenir une huile ou bien par extraction avec un solvant organique (par ex. alcool en C₁ à C₄, acétone, CO₂ supercritique avec un co-solvant de type éthanol ou acétone, etc.), plus particulièrement avec de l'acétone. Le solvant d'extraction est de préférence éliminé partiellement ou totalement après extraction.

Les galactolipides selon l'invention peuvent ensuite être séparés par distillation moléculaire, par chromatographie sur gel de silice greffée ou non, par chromatographie de partage centrifuge, par chromatographie à contre-courant, par séparation liquide / liquide ou tout autre moyen de purification bien connu de l'homme du métier. Les chromatographies peuvent utiliser un fluide supercritique ou un solvant organique (qui peut être sous forme d'un mélange de solvants organiques).

La présente invention a également pour objet une composition dermo-cosmétique ou dermatologique, comprenant au moins un galactolipide de formule (I) tel que défini ci-dessus et au moins un excipient physiologiquement acceptable, pour son utilisation dans la cicatrisation, de préférence de la peau; la réparation, le renforcement et le rééquilibre de la barrière cutanée dont le renforcement et le rééquilibre de la barrière mécanique de la peau ; l'hydratation cutanée, en particulier l'hydratation cutanée du stratum corneum ; et la prévention et le traitement des signes du vieillissement cutané, en particulier la prévention et le traitement du photovieillissement cutané.

Dans la présente invention, on entend désigner par « physiologiquement acceptable » ce qui est utile dans la préparation d'une composition dermo-cosmétique ou dermatologique, qui est généralement sûr, non toxique et ni biologiquement ni autrement non souhaitable et qui est acceptable pour une utilisation chez l'animal, incluant l'homme, en particulier par voie topique, notamment sur la peau.

La présente invention concerne également l'utilisation d'une composition dermo-cosmétique ou dermatologique, telle que définie ci-dessus, pour la cicatrisation, de préférence de la peau, et la prévention et le traitement du vieillissement cutané.

La présente invention concerne également une méthode de cicatrisation, notamment de la peau; la réparation, le renforcement et le rééquilibre de la barrière cutanée dont le renforcement et le rééquilibre de la barrière mécanique de la peau ; l'hydratation cutanée, en particulier l'hydratation cutanée du stratum corneum ; ou de prévention ou de traitement du vieillissement cutané, en particulier la prévention et le traitement du photovieillissement cutané ; comprenant l'administration à une personne en ayant besoin d'une quantité efficace d'une composition dermo-cosmétique ou dermatologique, telle que définie ci-dessus.

Une telle composition dermo-cosmétique ou dermatologique est destinée plus particulièrement à être appliquée de manière topique, en particulier sur la peau, et plus particulièrement sur une peau fragile.

Les compositions selon l'invention pourront se présenter sous les formes qui sont habituellement connues pour une administration topique, c'est à dire notamment les lotions, les mousses, les gels, les dispersions, les émulsions, les shampooings, les sprays, les sérums, les masques, les laits corporels ou les crèmes, avec des excipients permettant notamment une pénétration cutanée afin d'améliorer les propriétés et l'accessibilité du principe actif. Avantageusement, il s'agira d'une crème.

Ces compositions contiennent généralement, outre le ou les galactolipides selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants ou des eaux thermales, etc.

Ces compositions peuvent en outre contenir d'autres principes actifs conduisant à un effet complémentaire ou éventuellement synergique.

Avantageusement, les compositions selon la présente invention comprendront de 0,005 à 10% en poids, de préférence de 0,01% à 1% en poids, du au moins un galactolipide de formule (I) par rapport au poids total de la composition.

De préférence, les compositions selon la présente invention comprendront un mélange de galactolipides de formule (I).

Ces galactolipides selon l'invention pourront être introduits dans la composition sous une forme purifiée ou sous forme d'extrait. Dans le cas d'un extrait, il s'agira plus particulièrement d'un extrait de graines d'avoine ou de graines d'avoine maltée pouvant être obtenu notamment comme décrit précédemment.

La présente invention a également pour objet un galactolipide de formule (I') suivante : 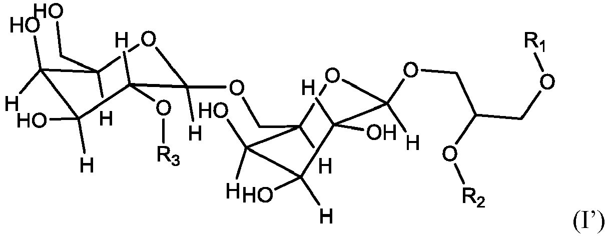 dans laquelle :
- R₁ et R₂, identiques ou différents, représentent un groupe -CO-R₄, 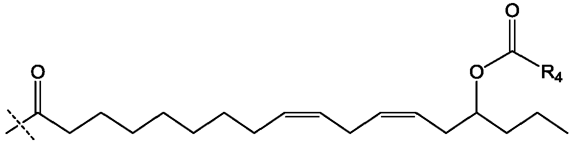 ou 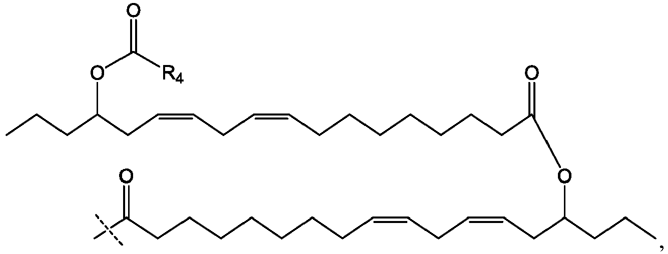
- R₃ représente un groupe -CO-R₅, et
- R₄ et R₅, identiques ou différents, représentent une chaîne hydrocarbonée linéaire ou ramifiée, de préférence linéaire, saturée ou insaturée, comprenant 9 à 25, notamment 11 à 21, en particulier 13 à 19, plus particulièrement 15 à 17, atomes de carbone, éventuellement substituée par un groupe OH.

Selon un mode de réalisation particulier, R₁ et R₂, identiques ou différents, représentent un groupe -CO-R₄ ou

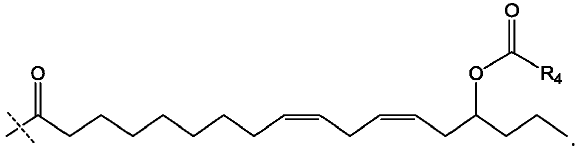

Le galactolipide de formule (I') pourra être plus particulièrement un galactolipide selon l'une des formules (Ic) et (Id) ci-dessous : 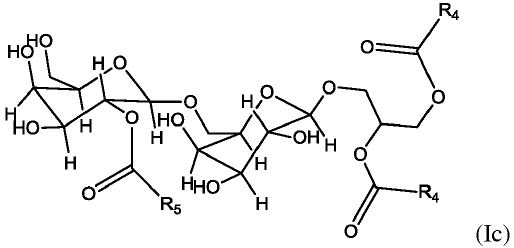 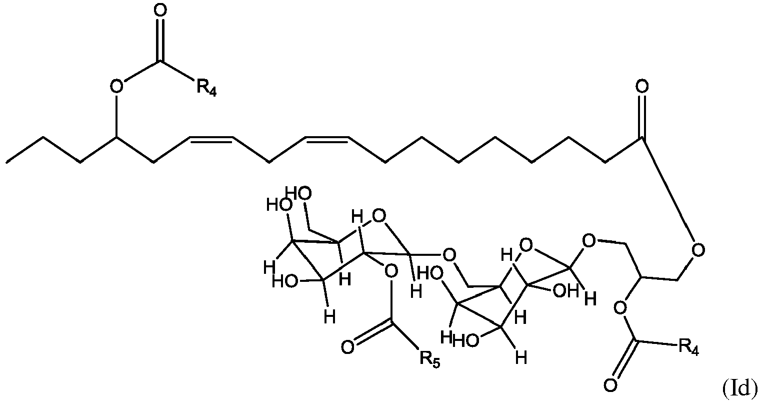 pour lesquelles R₄ et R₅ sont tels que définis ci-dessus et ci-après.

R₄ et R₅, identiques ou différents, représentent une chaîne hydrocarbonée linéaire ou ramifiée, de préférence linéaire, saturée ou insaturée, comprenant 9 à 25, notamment 11 à 21, en particulier 13 à 19, plus particulièrement 15 à 17, encore plus particulièrement 15 ou 17 atomes de carbone, éventuellement substituée par un groupe OH, et notamment non substituée.

Avantageusement, R₄ et R₅, identiques ou différents, représentent une chaîne hydrocarbonée linéaire ou ramifiée, de préférence linéaire, saturée ou comprenant 1 à 5, notamment 1 à 3 doubles liaisons C=C, comprenant 9 à 25, notamment 11 à 21, en particulier 13 à 19, plus particulièrement 15 à 17, encore plus particulièrement 15 ou 17 atomes de carbone, éventuellement substituée par un groupe OH, et notamment non substituée.

De préférence, R₄ et R₅, identiques ou différents, représentent une chaîne hydrocarbonée linéaire, saturée ou comprenant 1 à 3 (en particulier 1 ou 2) doubles liaisons C=C, comprenant 13 à 21, notamment 13 à 19, en particulier 15 à 17, plus particulièrement 15 ou 17, atomes de carbone, éventuellement substituée par un groupe OH, et notamment non substituée.

Plus particulièrement, les groupes R₄ et R₅, identiques ou différents, pourront être choisis parmi les groupes (C15:0), (C17:0), (C17:1), (C17:2), (C17:3) et (C17:2OH), plus particulièrement parmi les groupes (C15:0), (C17:0), (C17:1), (C17:2) et (C17:3) ; et encore plus particulièrement parmi les groupes (C15:0), (C17:0), (C17:1) et (C17:2).

De manière avantageuse, R₄ et R₅ sont identiques.

Le galactolipide selon l'invention pourra être choisi en particulier parmi les galactolipides selon les formules (Ic) et (Id) ci-dessus avec R₄ et R₅ identiques et choisis parmi les groupes (C15:0), (C17:0), (C17:1), (C17:2), (C17:3) et (C17:2OH), plus particulièrement parmi les groupes (C15:0), (C17:0), (C17:1), (C17:2) et (C17:3) ; et encore plus particulièrement parmi les groupes (C15:0), (C17:0), (C17:1) et (C17:2), tel que (C17:2).

Ces galactolipides de formule (I') peuvent être obtenus comme décrit précédemment pour les galactolipides de formule (I).

La présente invention a également pour objet un galactolipide de formule (I') pour son utilisation comme médicament.

La présente invention concerne également l'utilisation d'un galactolipide de formule (I') pour la préparation d'une composition dermo-cosmétique ou dermatologique.

La présente invention a également pour objet une composition dermo-cosmétique ou dermatologique comprenant au moins un galactolipide de formule (I') tel que défini ci-dessus et au moins un excipient physiologiquement acceptable.

Une telle composition est destinée plus particulièrement à être appliquée de manière topique, en particulier sur la peau, et plus particulièrement sur une peau fragile.

De telles compositions pourront se présenter sous les formes qui sont habituellement connues pour une administration topique, c'est à dire notamment les lotions, les mousses, les gels, les dispersions, les émulsions, les shampooings, les sprays, les sérums, les masques, les laits corporels ou les crèmes, avec des excipients permettant notamment une pénétration cutanée afin d'améliorer les propriétés et l'accessibilité du principe actif. Avantageusement, il s'agira d'une crème.

Ces compositions contiennent généralement, outre le ou les galactolipides selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants ou des eaux thermales, etc.

Ces compositions peuvent en outre contenir d'autres principes actifs conduisant à un effet complémentaire ou éventuellement synergique, et notamment d'autres galactolipides.

Avantageusement, les compositions selon la présente invention comprendront de 0,005 à 10% en poids, de préférence de 0,01% à 1% en poids, du au moins un galactolipide de formule (I') par rapport au poids total de la composition.

De préférence, les compositions selon la présente invention comprendront un mélange de galactolipides de formule (I').

Ces galactolipides selon l'invention pourront être introduits dans la composition sous une forme purifiée ou sous forme d'extrait. Dans le cas d'un extrait, il s'agira plus particulièrement d'un extrait de graines d'avoine ou de graines d'avoine maltée pouvant être obtenu notamment comme décrit précédemment.

La présente invention sera mieux comprise à la lumière des exemples non limitatifs qui suivent.

### FIGURES

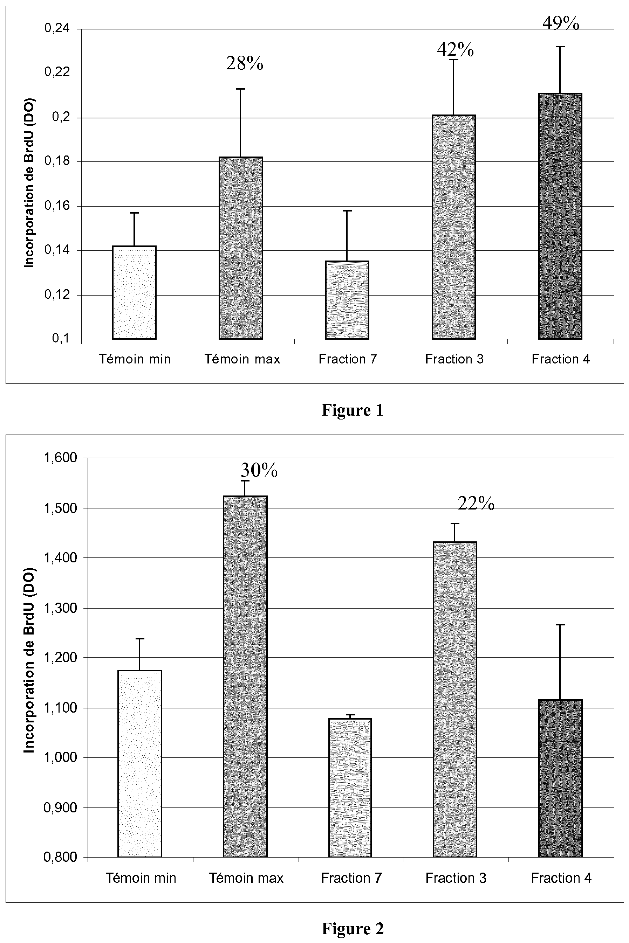

La **Figure 1** présente les résultats obtenus après 48 h d'incubation en termes d'incorporation de BrdU (DO) dans le cadre de contrôles (« témoin min » et « témoin max ») ou de l'addition de différents extraits (fractions 3, 4 et 7) sur des kératinocytes normaux humains (expérience représentative de 3 expériences indépendantes).
La **Figure 2** présente les résultats obtenus après 48 h d'incubation en termes d'incorporation de BrdU (DO) dans le cadre de contrôles (« témoin min » et « témoin max ») ou de l'addition de différents extraits (fractions 3, 4 et 7) sur des fibroblastes normaux humains (expérience représentative de 3 expériences indépendantes).

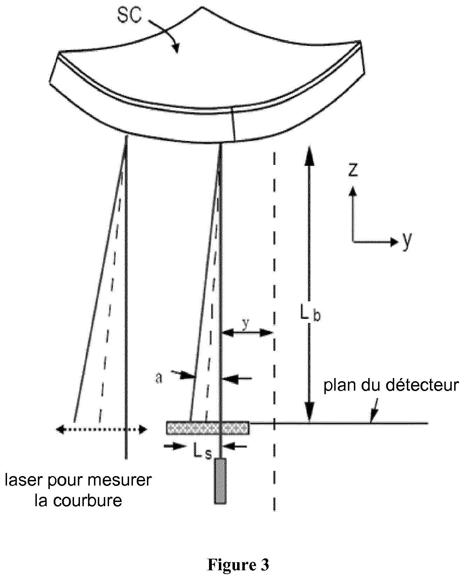

La **Figure 3** présente le principe de mesure de l'évolution des contraintes dans le modèle *in vitro* de SC isolé déshydraté.

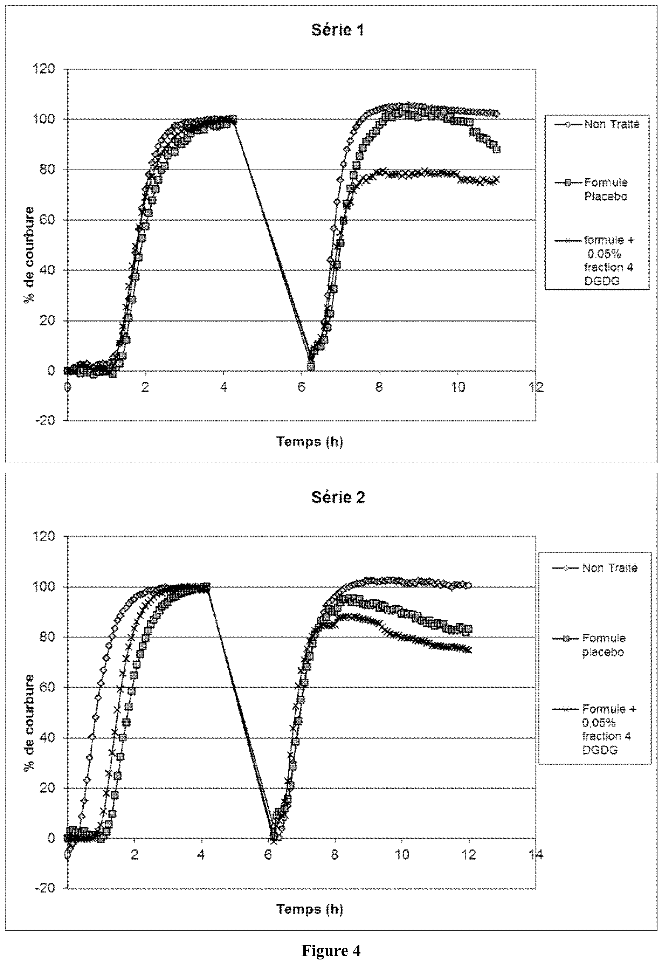

La **Figure 4** présente les résultats obtenus sur la protection de la barrière mécanique du stratum corneum humain issu de deux donneurs (séries 1 et 2) soumis à une déshydratation sans traitement ou après traitement avec une formulation contenant la fraction 4 ou ne la contenant pas (placebo).

### EXEMPLES

### Exemple 1 : Préparation d'une huile d'avoine maltée

Extraire 1kg de grains d'avoine maltés broyés par 7L d'acétone. Après séparation solide / liquide, l'acétone est éliminé par évaporation. L'extrait est obtenu, sous forme d'une huile jaune légèrement trouble. Cette huile contient après séparation sur colonne de silice 13% de lipides polaires dont 5% de DGDG et 4,5% de DGDG estolides.

### Exemple 2 : Préparation d'une fraction enrichie en acyl-DGDG et acyl-DGDG-estolides

Procéder à une extraction liquide / liquide de 500g d'huile d'avoine maltée dans un mélange heptane (2L) / éthanol (2L) / eau (0,2L). Laver plusieurs fois la phase supérieure contenant les triglycérides et la phase inférieure contenant les lipides polaires. Mettre à sec la phase inférieure et la déposer sur une colonne de silice puis éluer avec du chloroforme, puis un mélange chloroforme / méthanol 10% à 50% et enfin un mélange méthanol / acétone 50 : 50. Après analyse par chromatographie sur couche mince (CCM), regrouper les fractions de profil équivalent. 10 fractions ont été obtenues après regroupement. La fraction 3 éluée par le mélange CHCl₃/MeOH 90 : 10 contient majoritairement les acyl-DGDG et acyl-DGDG-estolides.

Les acylDGDG et acylDGDG estolides ont été purifiés et analysés par chromatographie de partage centrifuge. Les données analytiques des molécules pures sont présentées ci-dessous.

### Molécule acylDGDG :

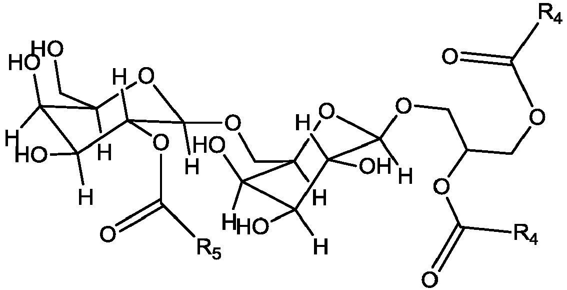

R₄ = R₅ = C17:2
Formule brute : C₆₉H₁₁₈O₁₆ (C18:2)2 / Glycérol / (Galactose)2 C18:2 Ion moléculaire à m/z = 1225.5 [1202.5+Na]⁺

Pics principaux du spectre RMN ¹³C :

| **Groupe** | **N°** | **13C (ppm)** |
|---|---|---|
| **Galactose 1** | 1 | 103.5 |
| | 2 | 73.3 |
| | 3 | 71.3 |
| | 4 | 68.7 |
| | 5 | 73.2 |
| | 6 | 67.1 |
| **Galactose 2** | 1 | 96.9 |
| | 2 | 71.2 |
| | 3 | 68.1 |
| | 4 | 70.3 |
| | 5 | 69.8 |
| | 6 | 62.6 |
| **Glycerol** | 1 | 62.4 |
| | 2 | 69.9 |
| | 3 | 67.9 |
| | C=O R1 | 173.7 |
| | C=O R2 | 173.4 |
| | C=O R3 | 174.2 |

### Molécule acylDGDG estolides :

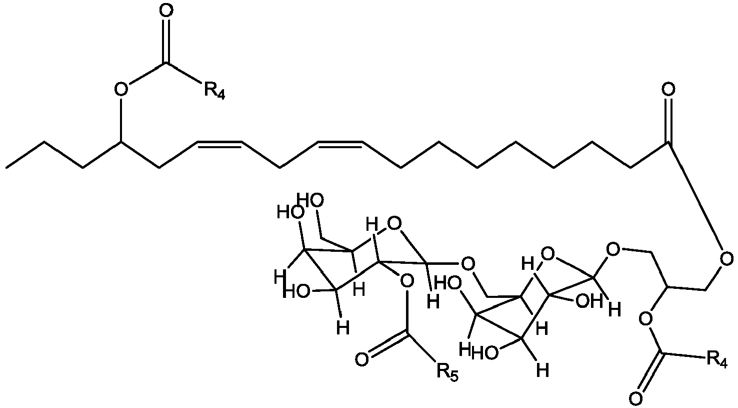

R₄ = R₅ = C17:2
Formule brute : C₈₇H₁₅₀O₁₈ (C18:2)3 / Glycérol / (Galactose)2 C18:2 Ion moléculaire à m/z = 1506.0 [1483.0+Na]⁺

### Exemple 3 : Préparation d'une fraction enrichie en DGDG-estolides

Procéder de la même manière que l'exemple 2. La fraction 4 éluée par le mélange CHCl₃/MeOH 90 : 10 contient majoritairement les DGDG monoestolides et DGDG diestolides, en particulier de formule (1) ou (2) ci-dessous. 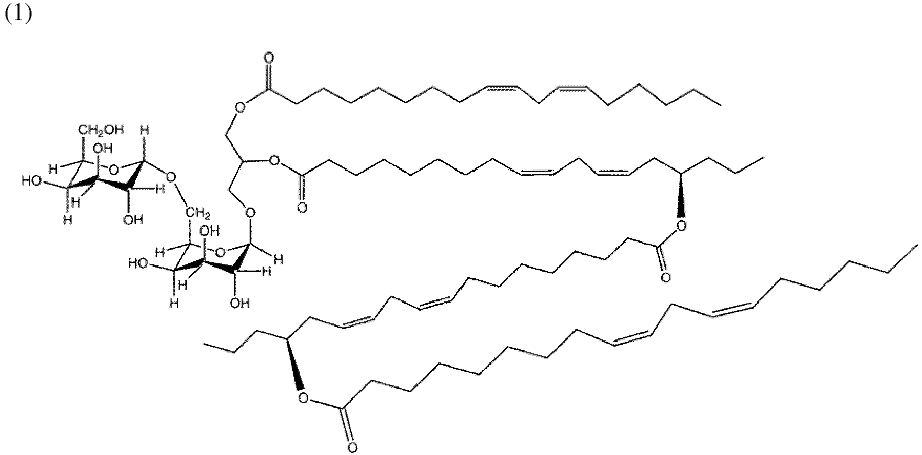 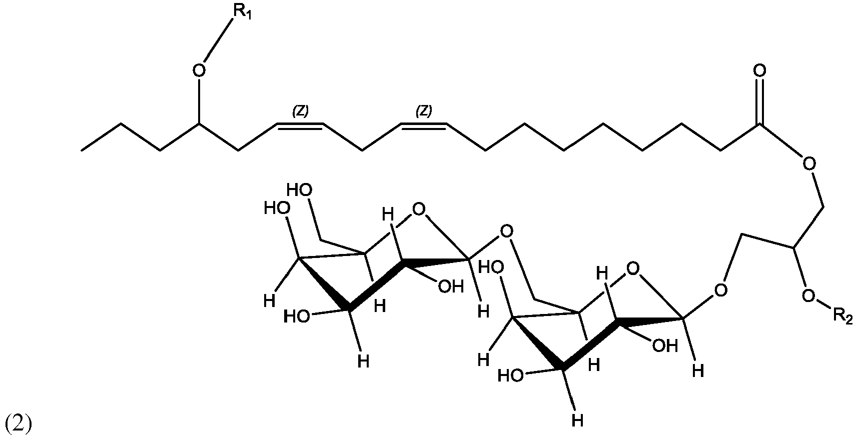 R₁ = R₂ = C15 :0, C17 :0, C17 :1 ou C17 :2

### Exemple 4 : Préparation d'une fraction de DGDG

Procéder de la même manière que l'exemple 2. La fraction 7 éluée avec le mélange CHCl₃/MeOH 85 : 15 contient en grande majorité les DGDG de l'huile d'avoine, en particulier de formule (3) ci-dessous. 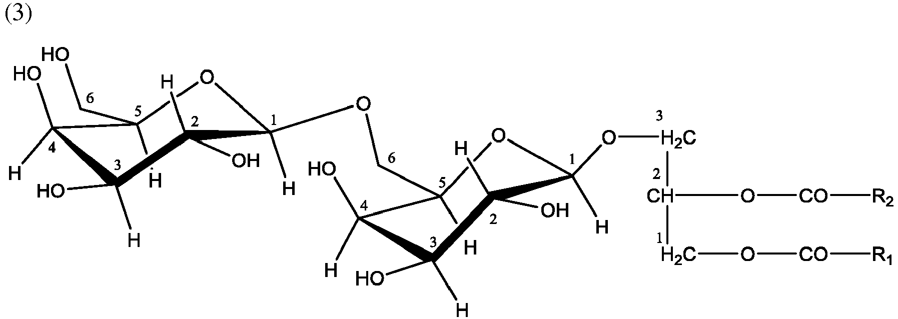 avec R₁ = R₂ = C15 :0, C17 :0, C17 :1 ou C17 :2

### EVALUATION pharmacologique

### Protocole

### Protocole :

La prolifération cellulaire est analysée par la technique d'incorporation d'un nucléotide, la 5-bromo-2'-déoxyuridine (BrdU), analogue de la thymidine, dans l'ADN des cellules en phase S, à 37°C. Le test mis en oeuvre est réalisé en plaque 96 puits.

La culture cellulaire a été réalisée à partir de kératinocytes normaux humains (KNH) ou de fibroblates normaux humains (FNH) issus d'explants de peau (abdominoplastie ou diminution mammaire). Les KNH ont été cultivés en condition stérile avec du milieu Keratinocyte-Serum Free Médium (K-SFM) (Gibco® - Life Technologies™) complémenté avec du Bovine Pituitary Extract (BPE) et de l'Epidermal Growth Factor Human recombinant (EGF) (Gibco®- Life Technologies™). Les FNH ont été cultivés en condition stérile avec du milieu DMEM (Dulbecco's Modified Eagle's Medium) complémenté avec 10% de sérum de veau foetal (SVF) (Gibco®- Life Technologies™). Les cellules sont d'abord privées de compléments ou sérum pendant 24h pour arrêter la croissance cellulaire avant de les incuber en présence des molécules à évaluer dans du KSFM sans complément (pour les KNH) ou dans le DMEM avec 3% de SVF (pour les FNH) pendant 48h à 37°C, dans une atmosphère à 5% de CO₂.

L'incorporation du BrdU, proportionnelle au taux de prolifération cellulaire, est évaluée par un système d'anticorps anti-BrdU couplés à la péroxydase. L'addition d'un substrat de la péroxydase développe une réaction colorée (kit de prolifération cellulaire Biotrak Elisa System). L'absorbance correspondante (DO) est mesurée à 450nm. Cette donnée est donc proportionnelle au taux de prolifération cellulaire.

### Analyse des résultats :

Les résultats sont exprimés:
- en DO (proportionnelle à l'incorporation de BrdU et donc au taux de prolifération cellulaire),
- en pourcentage de stimulation par rapport au témoin : ((DO traité / DO témoin) x 100) -100.

### Analyses statistiques :

Des analyses statistiques par le test de Dunnett ont été réalisées sur les valeurs brutes de la prolifération par rapport au témoin sans complément.

Ce test donne alors les valeurs des « p value » caractérisant la significativité des résultats obtenus pour les différentes conditions. Le degré de significativité est établi comme suit :
significatif pour p < 0,05 (*)
très significatif pour p < 0,01 (**)
hautement significatif pour p<0.001 (***)
non significatif pour p > 0,05

### Effet sur la prolifération des kératinocytes

Les résultats obtenus sont présentés sur la Figure 1.

Le contrôle « témoin min » est le contrôle non traité et sans complément EGF et BPE. Ces cellules prolifèrent peu.

Le contrôle « témoin max » a été incubé avec les compléments EGF (0,2 ng/ml) et BPE (25 µg/ml). Cette condition est un contrôle positif d'induction de la prolifération des kératinocytes. Il a induit la prolifération des kératinocytes de façon statistiquement significative.

La fraction 7 contenant les DGDG de l'huile d'avoine maltée n'a pas d'effet sur la prolifération des kératinocytes aux deux concentrations évaluées (1 et 5 µg/ml) (résultats montrés à 5 µg/ml).

Les fractions 3 et 4 (acylDGDG, acylDGDG-estolides et DGDG mono- et di-estolides) à 5 µg/ml ont induit la prolifération des kératinocytes de façon reproductible et statistiquement significative.

En induisant la prolifération des kératinocytes, les galactolipides de type DGDG selon l'invention favorisent la réparation et la cicatrisation cutanée, et en particulier la réparation épidermique. Ainsi, ils favorisent aussi le renforcement et le rééquilibre de la barrière cutanée.

### Effet sur la prolifération des fibroblastes

Les résultats obtenus sont présentés sur la Figure 2.

Le contrôle « témoin min » est le contrôle non traité incubé avec 3% de SVF. Ces cellules prolifèrent peu.

Le contrôle « témoin max » a été incubé avec 10% de SVF. Cette condition est un contrôle positif d'induction de la prolifération des fibroblastes. Il a induit la prolifération des fibroblastes de façon statistiquement significative.

Les DGDG (fraction 7) et les DGDG mono- et di-estolides (fraction 4) n'ont pas induit la prolifération des fibroblastes. En revanche, la fraction 3 (acylDGDG et acylDGDG-estolides) a induit la prolifération des fibroblastes de façon statistiquement significative. En induisant la prolifération des fibroblastes, les galactolipides de type acylDGDG favorisent la cicatrisation cutanée, en particulier la réparation dermique.

En induisant la prolifération des fibroblastes, les galactolipides de type acylDGDG favorisent aussi la prévention et le traitement du vieillissement cutané, et en particulier la prévention et le traitement du photovieillissement cutané.

### Effet sur la barrière mécanique et sur la protection à la déshydratation

### Protocole

Du stratum corneum (SC) issu d'explants de peau humaine a été isolé. Le SC est hydraté par immersion dans l'eau puis placé sur une lamelle de verre revêtue d'une feuille d'or réflectrice. Le SC hydraté et adhérant à la lamelle est placé dans une atmosphère à un taux d'hygrométrie de 10% pendant plusieurs heures. Le SC subit ainsi une déshydratation progressive, pendant laquelle des mesures de courbure de la lamelle sont réalisées toutes les 5 minutes à l'aide d'un laser réfléchi par la feuille d'or sur un capteur (cf. Figure 3). La courbure de l'ensemble SC + lamelle de verre est directement reliée à la tension et l'hydratation du SC, c'est à dire à l'état de la barrière mécanique du stratum corneum dans son ensemble.

Après cette première cinétique de déshydratation témoin, l'ensemble SC + lamelle est réhydraté dans une chambre humide pendant 2 heures pour un retour à la courbure nulle initiale. Le SC hydraté est traité (traitement solide ou liquide) puis une deuxième cinétique de déshydratation dans les mêmes conditions est lancée. L'évolution de la courbure après traitement est comparée à la première cinétique sans traitement.

Un effet émollient ou de protection à la déshydratation se traduit par un profil différent où la courbure est plus faible (notamment au pic maximal de tension - contrainte maximale) avec ou sans relâchement de la tension du SC (diminution de la courbure après atteinte du pic maximal pour arriver à une contrainte minimale).

Une évolution significative d'un ou plusieurs des paramètres (contrainte maximale, contrainte minimale, etc.) indique que la barrière mécanique du SC a été renforcée et rééquilibrée.

### Résultats

Les résultats obtenus sur 2 donneurs de stratum corneum sont présentés sur la Figure 4 (séries 1 et 2). On observe que la fraction 4 à 0,05% a un effet reproductible sur la protection de la barrière mécanique du SC soumis à un stress de déshydratation : en effet, le pic maximal de tension atteint est nettement diminué par rapport au témoin déshydraté sans traitement ou avec le traitement placebo, et cet effet protecteur est maintenu au cours du temps de la déshydratation.

En diminuant l'état de contrainte du SC, les galactolipides de type DGDG selon l'invention favorisent le renforcement et le rééquilibre de la barrière mécanique ; ainsi que l'état d'hydratation de la peau, en particulier l'état d'hydratation du stratum corneum.

### REFERENCES BIBLIOGRAPHIQUES

Biniek et al. (2012). Solar UV radiation reduces the barrier function of human skin. PNAS 109(42):17111-6.
Doehlert et al. (2010), Polar lipids from oat kernels. Cereal Chemistry 87(5): 467-474. Hamberg et al. (1998), Isolation and structure of a new galactolipid in oat seeds. Lipids 33:355-363.
Hambert et al. (1996), 15(R)-Hydroxylinoleic acid, an oxylipin from oat seeds. Phytochemistry 42:729-732.
Levi et al. (2010) (a) Drying stress and damage processes in human stratum corneum. Dauskardt. International Journal of Cosmetic Science 2010, 32, 276-293 ; (b) Application of substrate curvature method to differentiate drying stresses in topical coatings and human stratum corneum. Dauskardt. International Journal of Cosmetic Science 2010, 32, 294-298.
Moreau et al. (2008), The identification of Mono- Di-, Tri- and Tetragalactosyl-diacylglycerols and their natural estolides in Oat kernels. Lipids 43:533-548.
Ramos e Silva et al. (2013), Effects of age (neonates and elderly) on skin barrier function. Exp. Dermatol. 22(5):329-35. WO96/25142.
Vyumvuhore et al. (2015), The relationship between water loss, mechanical stress, and molecular structure of human stratum corneum ex vivo. J Biophotonics. 2015 8(3):217-25.

## Revendications

1. Galactolipide de formule (I) suivante : 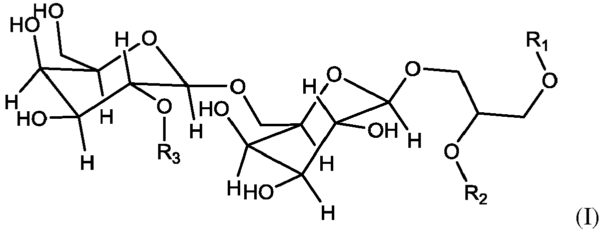 dans laquelle :
- R₁ et R₂, identiques ou différents, représentent un groupe -CO-R₄, 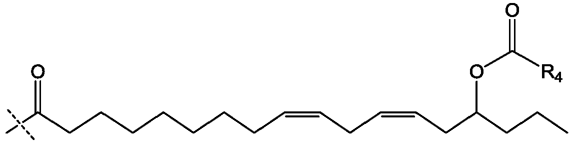 ou 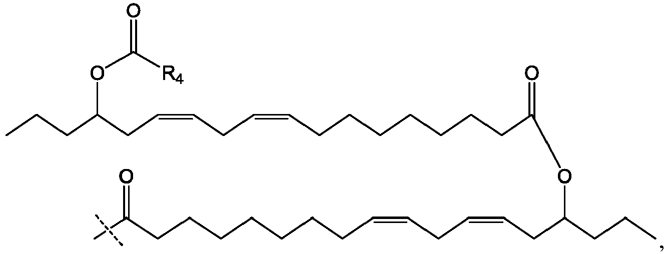
- R₃ représente :
▪ un groupe -CO-R₅ lorsque R₁ et R₂ représentent chacun, indépendamment l'un de l'autre, un groupe -CO-R₄,
▪ un atome d'hydrogène ou un groupe -CO-R₅ lorsqu'au moins R₁ ou R₂ représente un groupe autre que -CO-R₄, et
- R₄ et R₅, identiques ou différents, représentent une chaîne hydrocarbonée linéaire ou ramifiée, de préférence linéaire, saturée ou insaturée, comprenant 9 à 25, notamment 11 à 21, en particulier 13 à 19, plus particulièrement 15 à 17, atomes de carbone, éventuellement substituée par un groupe OH,
pour son utilisation dans la cicatrisation, de préférence de la peau, et dans la réparation, le renforcement et le rééquilibre de la barrière cutanée.

2. Utilisation d'un galactolipide de formule (I) telle que définie à la revendication 1, pour la prévention et le traitement du vieillissement cutané, en particulier pour la prévention et le traitement du photovieillissement cutané.

3. Galactolipide pour son utilisation selon la revendication 1 ou utilisation selon la revendication 2, **caractérisé en ce que** le galactolipide est un galactolipide selon l'une des formules (la) à (Id) suivantes : 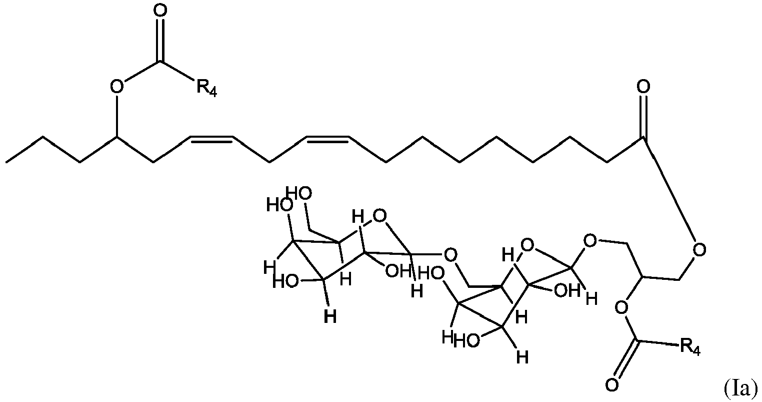 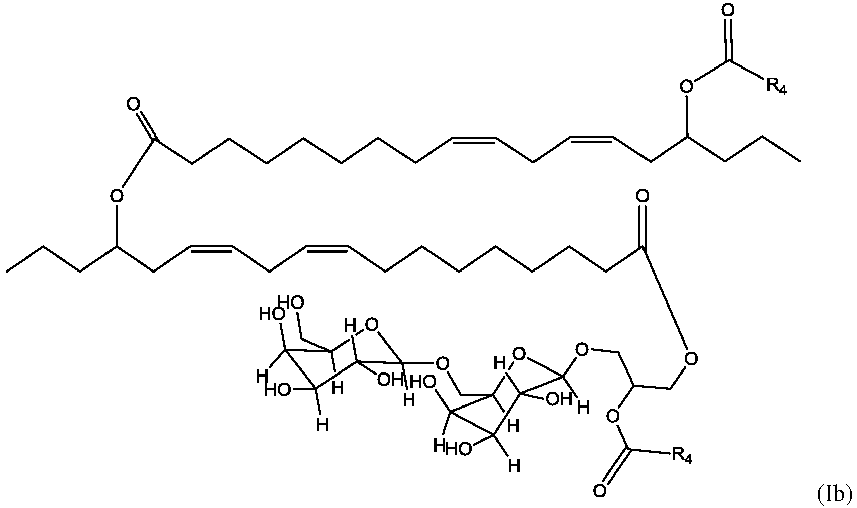 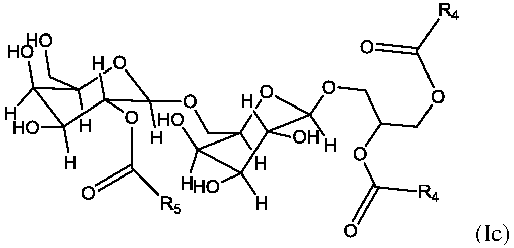 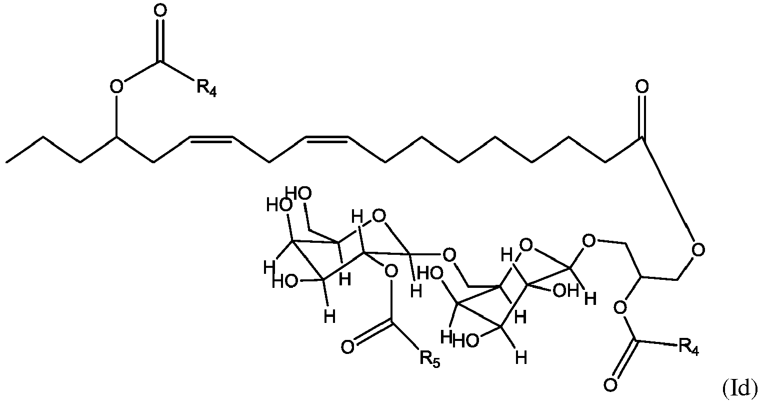 pour lesquelles R₄ et R₅ sont tels que définis à la revendication 1.

4. Galactolipide pour son utilisation selon la revendication 1 ou 3 ou utilisation selon la revendication 2 ou 3, **caractérisé en ce que** R₄ et R₅, identiques ou différents, représentent une chaîne hydrocarbonée linéaire, saturée ou comprenant 1 à 3 doubles liaisons C=C, comprenant 13 à 21, notamment 13 à 19, en particulier 15 à 17, plus particulièrement 15 ou 17, atomes de carbone, éventuellement substituée par un groupe OH.

5. Galactolipide pour son utilisation selon la revendication 1 ou 3 ou utilisation selon la revendication 2 ou 3, **caractérisé en ce que** R₄ et R₅, identiques ou différents, sont choisis parmi les groupes suivants : 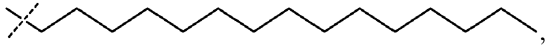 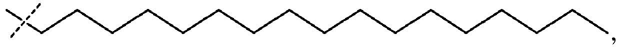 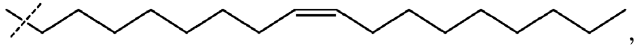 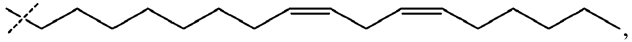 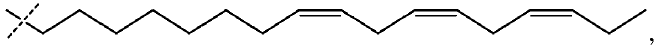 et

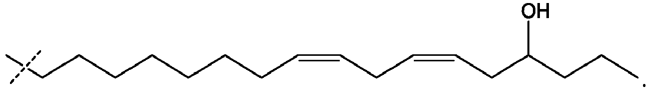

6. Galactolipide pour son utilisation selon l'une quelconque des revendications 1 et 3 à 5 ou utilisation selon l'une quelconque des revendications 2 à 5, **caractérisé en ce que** R₄ et R₅ sont identiques.

7. Composition dermatologique ou dermo-cosmétique comprenant au moins un galactolipide de formule (I) défini selon l'une quelconque des revendications 1 et 3 à 6 et au moins un excipient physiologiquement acceptable, pour son utilisation dans la cicatrisation, de préférence de la peau, et dans la réparation, le renforcement et le rééquilibre de la barrière cutanée.

8. Utilisation d'une composition dermatologique ou dermo-cosmétique comprenant au moins un galactolipide de formule (I) défini selon l'une quelconque des revendications 1 et 3 à 6 et au moins un excipient physiologiquement acceptable, pour la prévention et le traitement du vieillissement cutané, en particulier pour la prévention et le traitement du photovieillissement cutané.

9. Composition pour son utilisation selon la revendication 7 ou utilisation selon la revendication 8, **caractérisée en ce que** la composition dermatologique ou dermo-cosmétique est destinée à une application topique sur la peau.

10. Composition pour son utilisation selon la revendication 7 ou 9 ou utilisation selon la revendication 8 ou 9, **caractérisée en ce que** la composition dermatologique ou dermo-cosmétique comprend de 0,005 à 10% en poids, de préférence de 0,01% à 1% en poids, du au moins un galactolipide de formule (I) par rapport au poids total de la composition.

11. Galactolipide de formule (I') suivante : 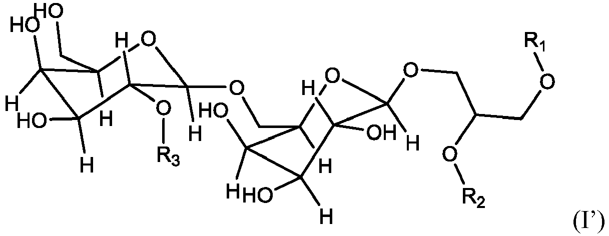 dans laquelle :
- R₁ et R₂, identiques ou différents, représentent un groupe -CO-R₄, 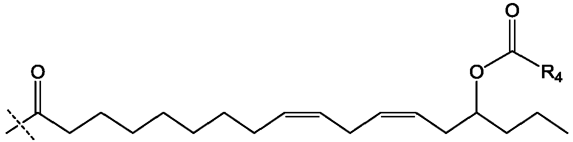 ou 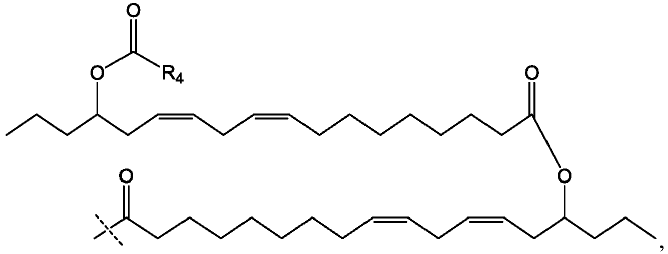
- R₃ représente un groupe -CO-R₅, et
- R₄ et R₅, identiques ou différents, représentent une chaîne hydrocarbonée linéaire ou ramifiée, de préférence linéaire, saturée ou insaturée, comprenant 9 à 25, notamment 11 à 21, en particulier 13 à 19, plus particulièrement 15 à 17, atomes de carbone, éventuellement substituée par un groupe OH.

12. Galactolipide selon la revendication 11, **caractérisé en ce qu'**il s'agit d'un galactolipide selon l'une des formules (Ic) et (Id) suivantes : 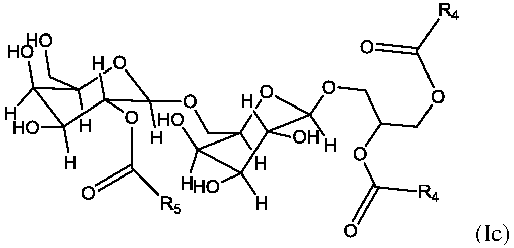 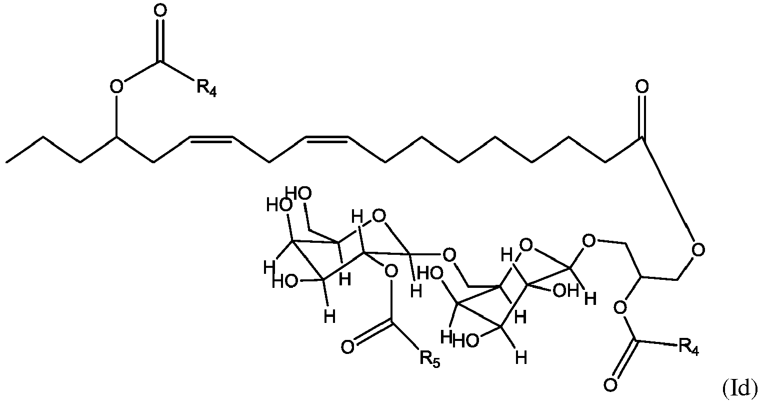 pour lesquelles R₄ et R₅ sont tels que définis à la revendication 11.

13. Galactolipide selon la revendication 11 ou 12, **caractérisé en ce que** R₄ et R₅, identiques ou différents, représentent une chaîne hydrocarbonée linéaire, saturée ou comprenant 1 à 3 doubles liaisons C=C, comprenant 13 à 21, notamment 13 à 19, en particulier 15 à 17, plus particulièrement 15 ou 17, atomes de carbone, éventuellement substituée par un groupe OH.

14. Galactolipide selon l'une quelconque des revendications 11 à 13, **caractérisé en ce que** R₄ et R₅, identiques ou différents, sont choisis parmi les groupes suivants : 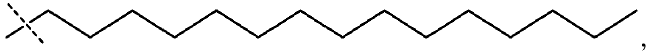 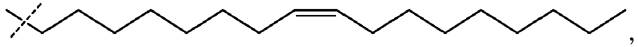 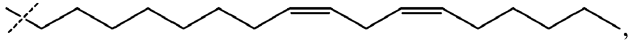 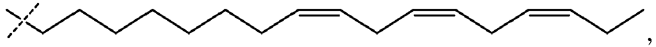 et

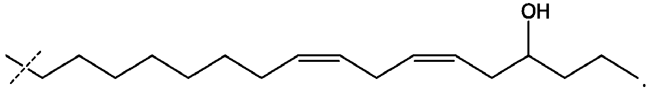

15. Galactolipide selon l'une quelconque des revendications 11 à 14, **caractérisé en ce que** R₄ et R₅ sont identiques.

16. Galactolipide selon l'une quelconque des revendications 11 à 15 pour son utilisation comme médicament.

17. Composition dermatologique ou dermo-cosmétique comprenant au moins un galactolipide selon l'une quelconque des revendications 11 à 15 et au moins un excipient physiologiquement acceptable.

## Patentansprüche

1. Galaktolipid der folgenden Formel (I): 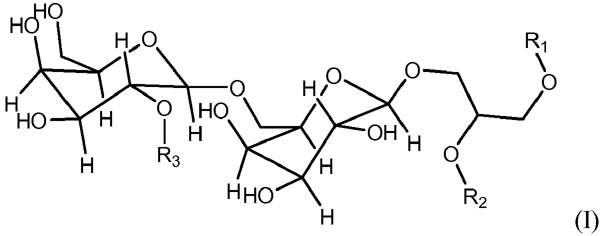 wobei:
- R₁ und R₂, identisch oder verschieden, für eine -CO-R₄-Gruppe stehen, 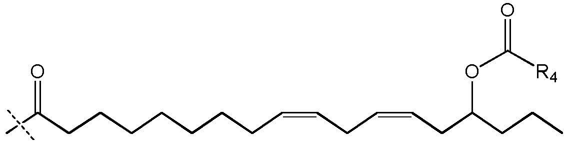 oder 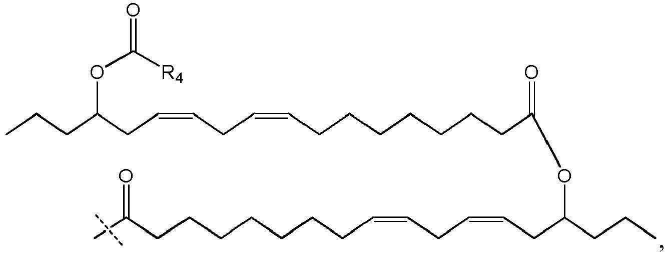
- R₃ steht für:
▪ eine -CO-R₅-Gruppe, wenn R₁ und R₂ jedes unabhängig voneinander für eine -CO-R₄-Gruppe stehen,
▪ ein Wasserstoffatom oder eine -CO-R₅-Gruppe, wenn mindestens R₁ oder R₂ für eine andere Gruppe als -CO-R₄ steht, und
- R₄ und R₅, identisch oder verschieden, für eine geradkettige oder verzweigte, vorzugsweise verzweigte, gesättigte oder ungesättigte Kohlenwasserstoffgruppe stehen, die 9 bis 25, vor allem 11 bis 21, insbesondere 13 bis 19, ganz besonders 15 bis 17 Kohlenstoffatome, gegebenenfalls durch eine OH-Gruppe substituiert, umfasst,
zu dessen Verwendung in der Wundheilung, vorzugsweise der Haut, und in der Reparatur, der Stärkung und der Wiederherstellung des Gleichgewichts der Hautbarriere.

2. Verwendung eines Galaktolipids der Formel (I) wie in Anspruch 1 definiert zur Vorbeugung und zur Behandlung der Hautalterung, insbesondere zur Vorbeugung und zur Behandlung der lichtbedingten Hautalterung.

3. Galaktolipid zu dessen Verwendung nach Anspruch 1, oder Verwendung nach Anspruch 2, **dadurch gekennzeichnet, dass** das Galaktolipid ein Galaktolipid nach einer der folgenden Formeln (Ia) bis (Id) ist: 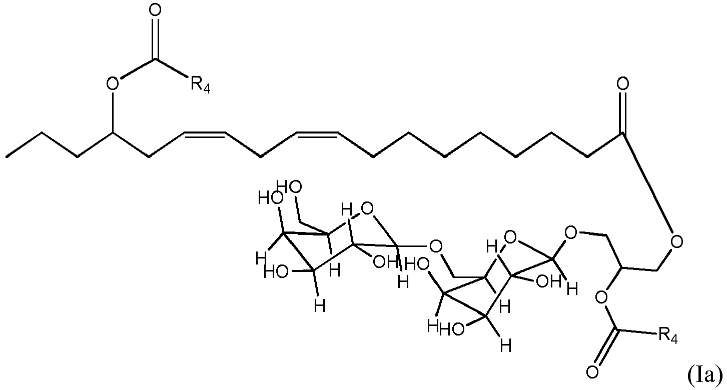 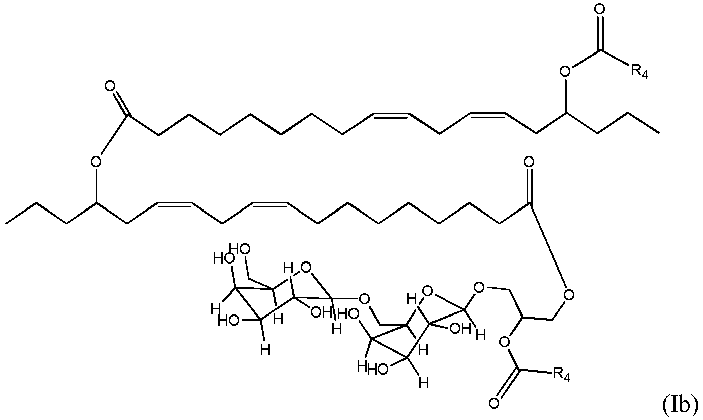 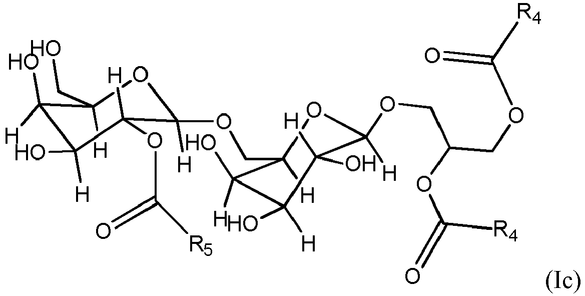 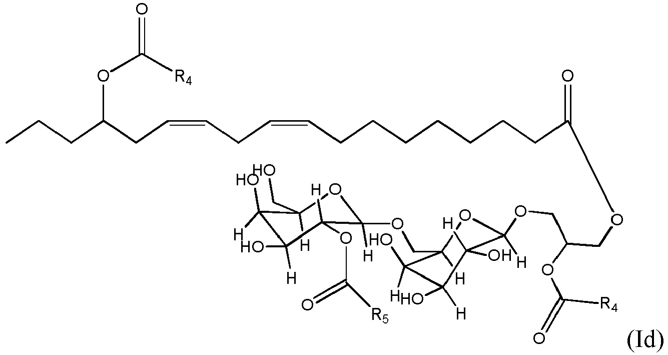 wobei R₄ und R₅ wie in Anspruch 1 definiert sind.

4. Galaktolipid zu dessen Verwendung nach Anspruch 1 oder 3, oder Verwendung nach Anspruch 2 oder 3, **dadurch gekennzeichnet, dass** R₄ und R₅, identisch oder verschieden, für eine geradkettige, gesättigte oder 1 bis 3 C=C-Doppelbildungen umfassende Kohlenwasserstoffkette stehen, die 13 bis 21, vor allem 13 bis 19, insbesondere 15 bis 17, ganz besonders 15 oder 17 Kohlenstoffatome, gegebenenfalls durch eine OH-Gruppe substituiert, umfasst.

5. Galaktolipid zu dessen Verwendung nach Anspruch 1 oder 3, oder Verwendung nach Anspruch 2 oder 3, **dadurch gekennzeichnet, dass** R₄ und R₅, identisch oder verschieden, aus den folgenden Gruppen ausgewählt sind: 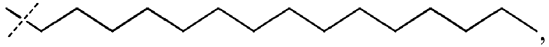 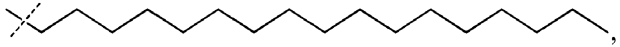 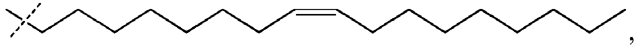 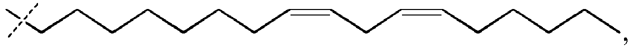 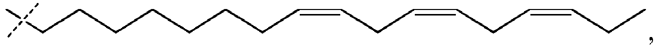 und

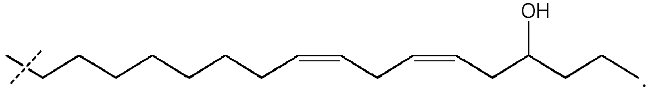

6. Galaktolipid zu dessen Verwendung nach einem der Ansprüche 1 und 3 bis 5, oder Verwendung nach einem der Ansprüche 2 bis 5, **dadurch gekennzeichnet, dass** R₄ und R₅ identisch sind.

7. Dermatologische oder dermokosmetische Zusammensetzung, die mindestens ein Galaktolipid der Formel (I), definiert nach einem der Ansprüche 1 und 3 bis 6, und mindestens einen physiologisch verträglichen Hilfsstoff umfasst, zu deren Verwendung in der Wundheilung, vorzugsweise der Haut, und in der Reparatur, der Stärkung und der Wiederherstellung des Gleichgewichts der Hautbarriere.

8. Verwendung einer dermatologischen oder dermokosmetischen Zusammensetzung, die mindestens ein Galaktolipid der Formel (I), definiert nach einem der Ansprüche 1 und 3 bis 6, und mindestens einen physiologisch verträglichen Hilfsstoff umfasst, zur Vorbeugung und zur Behandlung der Hautalterung, insbesondere zur Vorbeugung und zur Behandlung der lichtbedingten Hautalterung.

9. Zusammensetzung zu deren Verwendung nach Anspruch 7, oder Verwendung nach Anspruch 8, **dadurch gekennzeichnet, dass** die dermatologische oder dermokosmetische Zusammensetzung für eine topische Anwendung auf der Haut vorgesehen ist.

10. Zusammensetzung zu deren Verwendung nach Anspruch 7 oder 9, oder Verwendung nach Anspruch 8 oder 9, **dadurch gekennzeichnet, dass** die dermatologische oder dermokosmetische Zusammensetzung bezogen auf das Gesamtgewicht der Zusammensetzung 0,005 bis 10 Gewichts-%, vorzugsweise 0,01 Gewichts-% bis 1 Gewichts-% des mindestens einen Galaktolipids der Formel (I) umfasst.

11. Galaktolipid der folgenden Formel (I'): 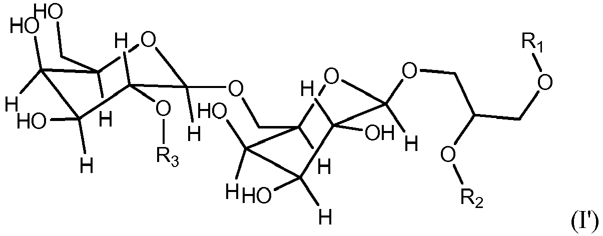 wobei:
- R₁ und R₂, identisch oder verschieden, für eine -CO-R₄-Gruppe stehen, 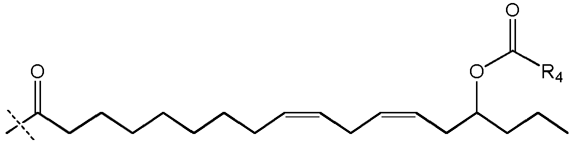 oder 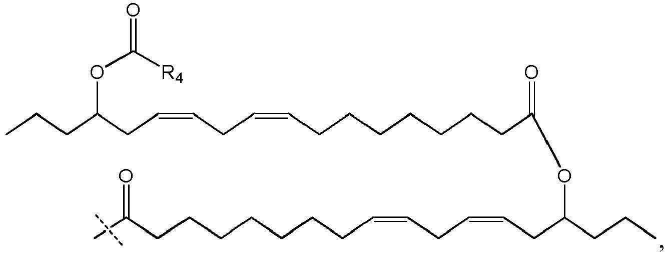
- R₃ für eine -CO-R₅-Gruppe steht, und
- R₄ und R₅, identisch oder verschieden, für eine geradkettige oder verzweigte, vorzugsweise geradkettige, gesättigte oder ungesättigte Kohlenwasserstoffkette stehen, die 9 bis 25, vor allem 11 bis 21, insbesondere 13 bis 19, ganz besonders 15 bis 17 Kohlenstoffatome, gegebenenfalls durch eine OH-Gruppe substituiert, umfasst.

12. Galaktolipid nach Anspruch 11, **dadurch gekennzeichnet, dass** es sich um ein Galaktolipid nach einer der folgenden Formeln (Ic) und (Id) handelt: 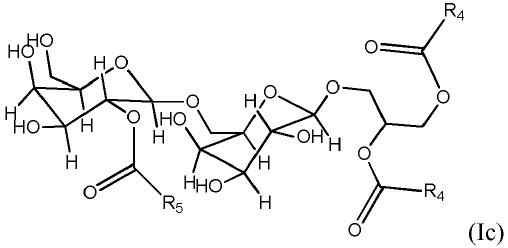 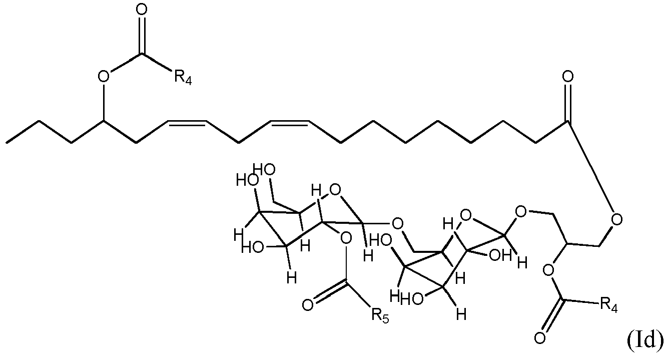 wobei R₄ und R₅ wie in Anspruch 11 definiert sind.

13. Galaktolipid nach Anspruch 11 oder 12, **dadurch gekennzeichnet, dass** R₄ und R₅, identisch oder verschieden, für eine geradkettige, gesättigte oder 1 bis 3 C=C-Dippelbindungen umfassende Kohlenwasserstoffkette stehen, die 13 bis 21, vor allem 13 bis 19, insbesondere 15 bis 17, ganz besonders 15 oder 17 Kohlenstoffatome, gegebenenfalls durch eine OH-Gruppe substituiert, umfasst.

14. Galaktolipid nach einem der Ansprüche 11 bis 13, **dadurch gekennzeichnet, dass** R₄ und R₅, identisch oder verschieden, aus den folgenden Gruppen ausgewählt sind: 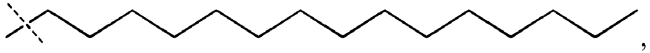 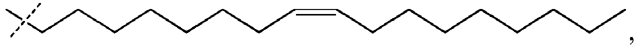 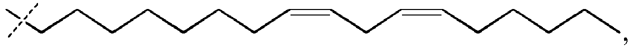 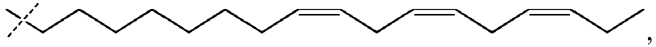 und

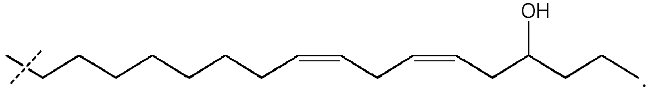

15. Galaktolipid nach einem der Ansprüche 11 bis 14, **dadurch gekennzeichnet, dass** R₄ und R₅ identisch sind.

16. Galaktolipid nach einem der Ansprüche 11 bis 15 zu dessen Verwendung als Arzneimittel.

17. Dermatologische oder dermokosmetische Zusammensetzung, die mindestens ein Galaktolipid nach einem der Ansprüche 11 bis 15, und mindestens einen physiologisch verträglichen Hilfsstoff umfasst.

## Claims

1. Galactolipid of the following formula (I): 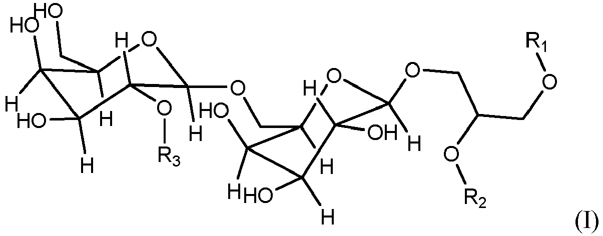 wherein:
- R₁ and R₂, identical or different, represent a group -CO-R₄, 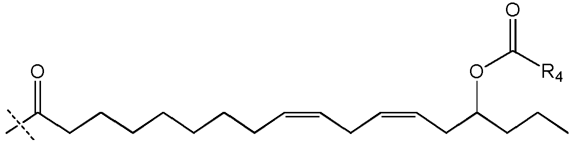 or 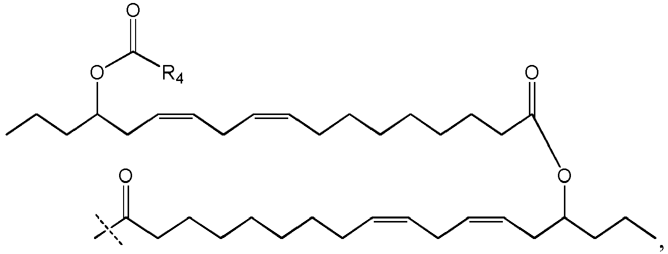
- R₃ represents:
▪ a group -CO-R₅ when R₁ and R₂ each represents, independently from each other, a group -CO-R₄,
▪ a hydrogen atom or a group -CO-R₅ when at least R₁ or R₂ represents a group other than -CO-R₄, and
- R₄ and R₅, identical or different, represent a linear or branched, preferably linear, saturated or unsaturated hydrocarbon chain comprising 9 to 25, more particularly 11 to 21, specifically 13 to 19, more specifically 15 to 17, carbon atoms, possibly substituted by an OH group,
for its use in the healing, preferably of the skin, and in the repair, the reinforcement and the rebalancing of the cutaneous barrier.

2. Use of a galactolipid of formula (I) as defined in claim 1, for the prevention and the treatment of cutaneous aging, more particularly for the prevention and the treatment of cutaneous photo-aging.

3. Galactolipid for its use according to claim 1 or use according to claim 2, **characterised in that** the galactolipid is a galactolipid according to one of the following formulae (Ia) to (Id): 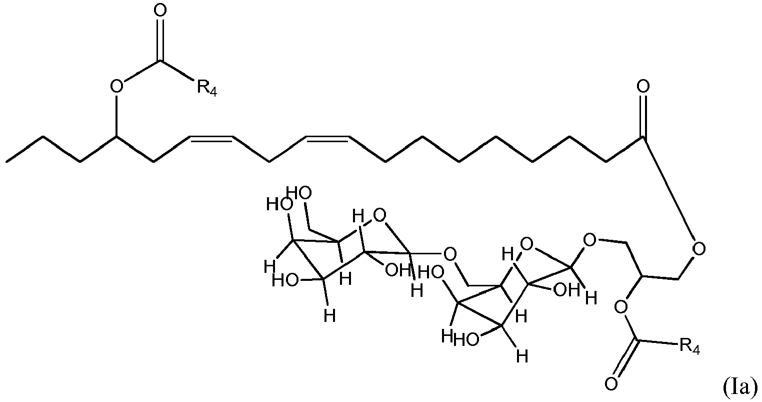 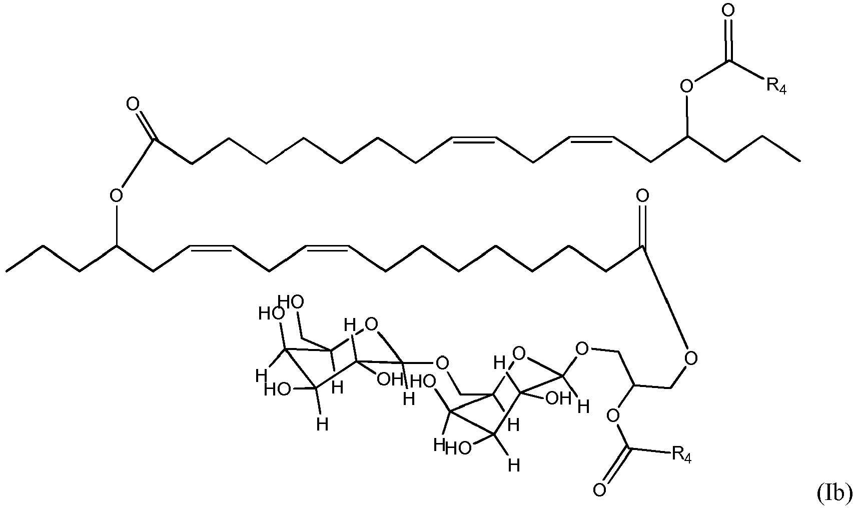 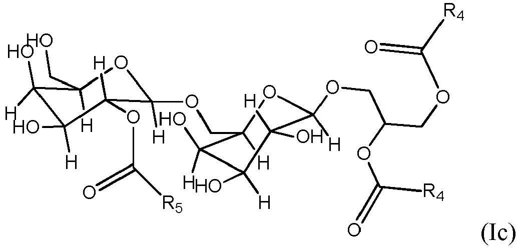 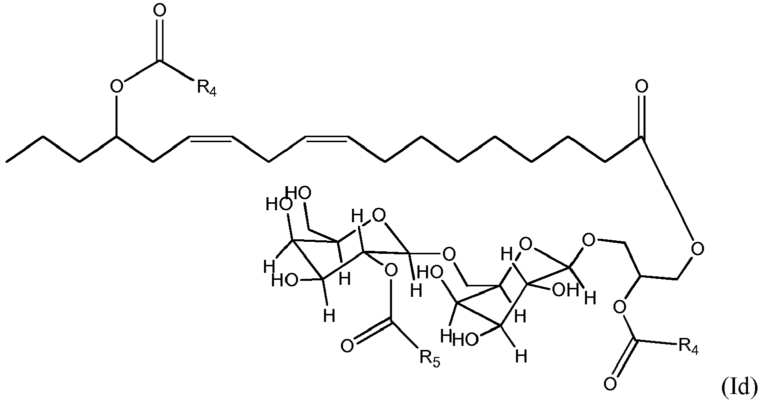 for which R₄ and R₅ are as defined in claim 1.

4. Galactolipid for its use according to claim 1 or 3 or use according to claim 2 or 3, **characterised in that** R₄ and R₅, identical or different, represent a linear hydrocarbon chain, saturated or comprising 1 to 3 double bonds C=C, comprising 13 to 21, more particularly 13 to 19, specifically 15 to 17, more specifically 15 or 17, carbon atoms, possibly substituted by an OH group.

5. Galactolipid for its use according to claim 1 or 3 or use according to claim 2 or 3, **characterised in that** R₄ and R₅, identical or different, are selected from amongst the following groups: 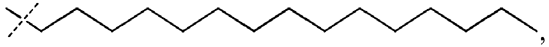 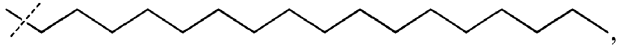 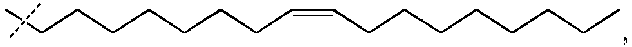 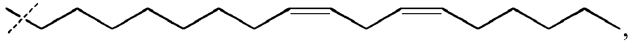 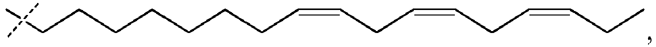 and

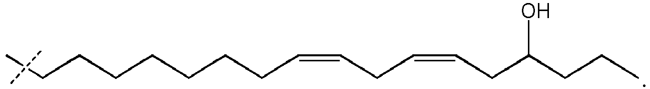

6. Galactolipid for its use according to any one of claims 1 and 3 to 5 or use according to any one of claims 2 to 5, **characterised in that** R₄ and R₅ are identical.

7. Dermatological or dermo-cosmetic composition comprising at least one galactolipid of formula (I) defined according to any one of claims 1 and 3 to 6 and at least one physiologically acceptable excipient, for its use in healing, preferably of the skin, and in the repair, the reinforcement and the rebalancing of the cutaneous barrier.

8. Use of a dermatological or dermo-cosmetic composition comprising at least one galactolipid of formula (I) defined according to any one of claims 1 and 3 to 6 and at least one physiologically acceptable excipient, for the prevention and the treatment of cutaneous aging, more particularly for the prevention and the treatment of cutaneous photo-aging.

9. Composition for its use according to claim 7 or use according to claim 8, **characterised in that** the dermatological or dermo-cosmetic composition is intended for the topical application on the skin.

10. Composition for its use according to claim 7 or 9 or use according to claim 8 or 9, **characterised in that** the dermatological or dermo-cosmetic composition comprises from 0.005 to 10% by weight, preferably from 0.01% to 1% by weight, of at least one galactolipid of formula (I) with respect to the total weight of the composition.

11. Galactolipid of the following formula (I'): 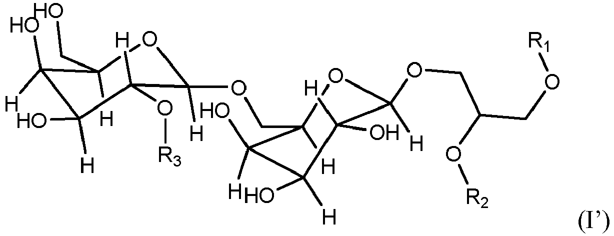 wherein:
- R₁ and R₂, identical or different, represent a group -CO-R₄, 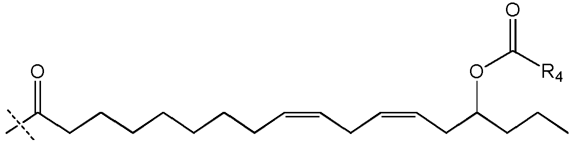 or 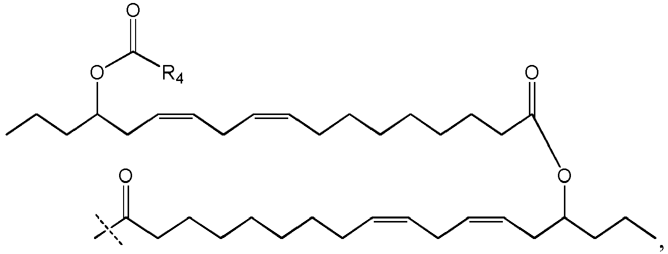
- R₃ represents a group -CO-R₅, and
- R₄ and R₅, identical or different, represent a linear or branched, preferably linear, saturated or unsaturated hydrocarbon chain comprising 9 to 25, more particularly 11 to 21, specifically 13 to 19, more specifically 15 to 17, carbon atoms, possibly substituted by an OH group.

12. Galactolipid according to claim 11, **characterised in that** it is a galactolipid according to one of the following formulae (Ic) and (Id): 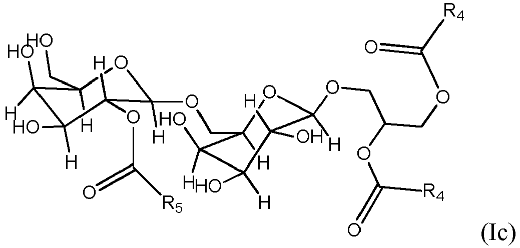 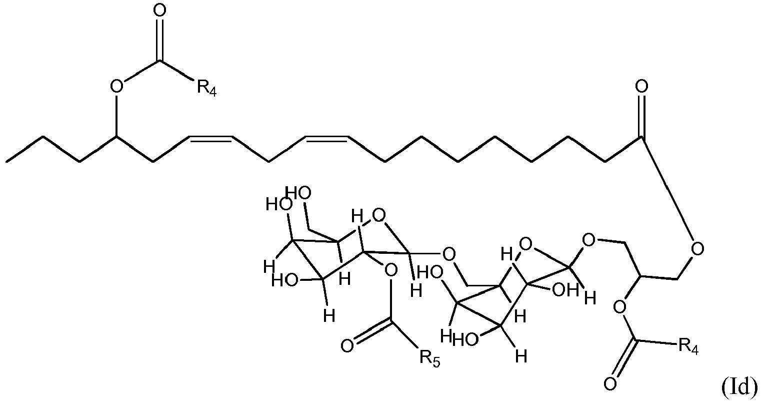 for which R₄ and R₅ are as defined in claim 11.

13. Galactolipid according to claim 11 or 12, **characterised in that** R₄ and R₅, identical or different, represent a linear hydrocarbon chain, saturated or comprising 1 to 3 double bonds C=C, comprising 13 to 21, more particularly 13 to 19, specifically 15 to 17, more specifically 15 or 17, carbon atoms, possibly substituted by an OH group.

14. Galactolipid according to any one of claims 11 to 13, **characterised in that** R₄ and R₅, identical or different, are selected from amongst the following groups: 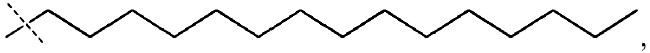 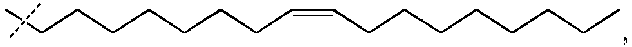 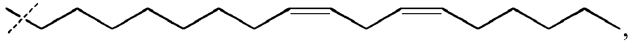 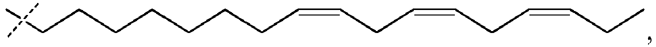 and

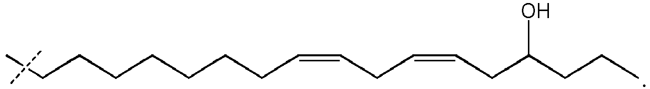

15. Galactolipid according to any one of claims 11 to 14, **characterised in that** R₄ and R₅ are identical.

16. Galactolipid according to any one of claims 11 to 15 for its use as a medication.

17. Dermatological or dermo-cosmetic composition comprising at least one galactolipid according to any one of claims 11 to 15 and at least one physiologically acceptable excipient.
